(19) Europäisches Patentamt
European Patent Office
Office européen des brevets

(11) Veröffentlichungsnummer: **0 182 232 B1**

(12) **EUROPÄISCHE PATENTSCHRIFT**

(45) Veröffentlichungstag der Patentschrift: **20.03.91**

(51) Int. Cl.$^5$: **A61K 7/06**

(21) Anmeldenummer: **85114283.6**

(22) Anmeldetag: **09.11.85**

(54) **Konditionierende Haarpflegemittel.**

(30) Priorität: **17.11.84 DE 3442175**

(43) Veröffentlichungstag der Anmeldung:
**28.05.86 Patentblatt 86/22**

(45) Bekanntmachung des Hinweises auf die Patenterteilung:
**20.03.91 Patentblatt 91/12**

(84) Benannte Vertragsstaaten:
**AT BE CH DE FR GB IT LI NL SE**

(56) Entgegenhaltungen:
**FR-A- 2 324 290**
**FR-A- 2 349 646**
**FR-A- 2 432 309**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Hensen, Hermann, Dr.**
**Tizianweg 4**
**W-4010 Hilden(DE)**
Erfinder: **Rutzen, Horst, Dr.**
**Falkenweg 12**
**W-4018 Langenfeld(DE)**
Erfinder: **Busch, Peter, Dr.**
**Gottfried-August-Bürger-Strasse 10**
**W-4006 Erkrath-Unterbach(DE)**
Erfinder: **Stuhrmann, Dagmar**
**Eichelstrasse 62**
**W-4000 Düsseldorf(DE)**
Erfinder: **Thiele, Klaus**
**Rügenweg 5**
**W-4018 Langenfeld(DE)**

**EP 0 182 232 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Haarpflegemittel mit konditionierender Wirkung, die quartäre Ammoniumverbindungen mit besonderen haarkosmetischen und verarbeitungstechnischen Eigenschaften enthalten.

Kationische Tenside vom Typ der quartären Ammoniumverbindungen werden in der Haarkosmetik als avivierende und konditionierende Wirkstoffe zur Verbesserung der Kämmbarkeit, der Fülle und des Griffs der Haare und zur Senkung der statischen Aufladbarkeit der Haare eingesetzt. Die Produkte werden meist in Haarnachbehandlungsmitteln eingesetzt, die nach der Haarwäsche dem Haar wieder günstige haarkosmetische Eigenschaften verleihen sollen. Sie eignen sich aber wenig als Zusatzmittel zu Haarwaschmitteln, um gleichzeitig mit der Wäsche einen gewissen konditionierenden Effekt zu erzielen, da die meisten bekannten quartären Ammoniumverbindungen mit den in Shampoos gebräuchlichen schaumstarken anionischen Tensiden in den für eine ausreichende konditionierende Wirkung erforderlichen Konzentrationen nicht verträglich sind, sondern schwer wasserlösliche und kosmetisch unwirksame Niederschläge bilden. Quartäre Ammoniumverbindungen, die mit Aniontensiden besser verträglich sind, weisen meist eine unbefriedigende konditionierende Wirkung auf.

Wasserlösliche kationische Polymere sind zwar meist mit anionischen Tensiden verträglich, haben aber andere Nachteile, z.B. Akkumulation auf dem Haar nach mehrmaliger Behandlung und die unzureichende Verminderung der statischen Aufladbarkeit des trockenen Haars.

Es bestand daher die Aufgabe, quartäre Ammoniumverbindungen zu finden, die starke avivierende und konditionierende Eigenschaften auf menschlichem Haar besitzen und die auch in Haarwaschmittel (Shampoos) auf Basis schaumstarker anionischer Tenside in Konzentrationen eingearbeitet werden können, die für eine befriedigende kosmetische Wirkung ausreichend sind ohne daß Trübungen und Ausfällungen auftreten.

Weiterhin ist es problematisch, avivierende und konditionierende Haarpflegemittel wie beispielsweise Haarspülungen mit einfachen Mitteln zu einer dem Verbraucher zusagenden Konsistenz zu verdicken.

Es wurde nun überraschenderweise gefunden, daß bestimmte quartäre Ammoniumverbindungen über stark avivierende und konditionierende Eigenschaften verfügen und gleichzeitig eine ausgezeichnete Verträglichkeit mit anionischen Tensiden aus den Klassen der Fettalkoholsulfate und Fettalkoholethersulfate aufweisen. Überdies sind wäßrige Lösungen dieser quartären Ammoniumverbindungen sehr gut mit einfachen, wasserlöslichen Salzen zu verdicken.

Gegenstand der Erfindung sind somit Haarpflegemittel mit einem Gehalt an quartären Ammoniumverbindungen, dadurch gekennzeichnet, daß als quartäre Ammoniumverbindungen solche der allgemeinen Formel (I)

$$\left[ R^1 - \underset{\displaystyle OH}{\underset{|}{CH}} - CH_2 - \overset{\displaystyle R^2}{\overset{|}{\underset{\displaystyle R^3}{\underset{|}{N^+}}}} - R^4 \right] \quad \frac{1}{n} \; A^{n(-)} \qquad (I),$$

in der $R^1$ eine n-Alkylgruppe mit 12 - 16 C-Atomen, $R^2$ eine n-Alkylgruppe mit 1 - 4 C-Atomen, $R^3$ eine Hydroxyalkylgruppe mit 2 - 4 C-Atomen und $R^4$ eine n-Alkylgruppe mit 1 - 4 C-Atomen oder eine Hydroxyalkylgruppe mit 2 - 4 C-Atomen, A ein anorganisches oder organisches Säureanion und n dessen Wertigkeit darstellen, enthalten sind und das Mittel neben üblichen Zusätzen solcher Mittel entweder

a) Aniontenside der allgemeinen Formel (II)

$$R^5\text{-}O(C_2H_4O)_n\text{-}SO_3\ M \qquad (II),$$

in der $R^5$ eine lineare Alkylgruppe mit 10 - 16 C-Atomen, n = 0 oder eine Zahl von 1 - 12, M ein Lithium-, Natrium-, Kalium-, Magnesium-, Ammonium-, Mono-, Di- oder Triethanolammonium darstellt,

oder

b) gelöste einfache Salze aus der Gruppe der Chloride, Bromide, Sulfate, Carbonate und/oder Phosphate des Lithiums, Natriums, Kaliums oder Magnesiums

enthält.

Aus der französischen Patentschrift FR-A-2 324 290 sind zwar Kombinationen von quartären Ammoniumverbindungen mit Fettalkoholsulfaten und Fettalkoholethersulfaten bekannt; die Druckschrift gibt jedoch keine Hinweise auf die besondere Eignung der quartären Ammoniumverbindungen der Formel (I) gemäß Anspruch 1. Vielmehr verdeutlichen die Beispiele der Druckschrift die Problematik der Kombinationen von quartären Ammoniumverbindungen mit anionischen Tensiden.

Weichmachungsmittel, die neben quartären Ammoniumverbindungen Salze enthalten, sind aus der französischen Patentschrift FR-A-2 349 646 bekannt. Dieser Druckschrift ist jedoch zu entnehmen, daß selbst geringe Mengen an Salzen die Viskosität dieser Mittel verringern und deren Dispersionszustand verschlechtern. Lediglich durch Zusatz spezieller Polyoxyalkylenderivate sind Mittel realisierbar, deren Viskosität und Wirksamkeit durch die Gegenwart wasserlöslicher Salze oder anderer Zusätze nicht nachteilig beeinflußt werden. Diese Druckschrift liefert somit keinerlei Hinweise auf die Verdickbarkeit wäßriger Lösungen der speziellen Ammoniumverbindungen der Formel (I) gemäß Anspruch 1 mit wasserlöslichen einfachen Salzen.

Die quartären Ammoniumverbindungen der allgemeinen Formel (I) sind bekannt. Ihre Herstellung aus Epoxyalkanen der allgemeinen Formel

$$R^1-CH-CH_2$$
\ /
O

und tertiären Aminsalzen der Formel

$$\left[R^2N^+HR^3R^4\right] \cdot \frac{1}{n} A^{n(-)}$$

wobei $R^1$, $R^2$ $R^3$ und $R^4$ und n die in Formel (I) angegebene Bedeutung haben, ist in DE-OS 31 16 087 beschrieben.

Bevorzugt geeignet zur Herstellung der erfindungsgemäßen Haarpflegemittel sind Verbindungen der Formel (I), in der $R^2$ eine Methylgruppe, $R^3$ eine 2-Hydroxyethylgruppe, $R^4$ eine Methyl- oder 2-Hydroxyethylgruppe und A ein Chloridion darstellen. Diese Verbindungen besitzen besonders günstige, die Kämmbarkeit des Haars verbessernde und die statische Aufladbarkeit verringende Eigenschaften. Sie können in Haarpflegemitteln in Mengen von 0,1 - 15 Gew.-% eingesetzt werden.

Bevorzugte, stark schäumende Aniontenside der allgemeinen Formel (II) sind Alkylsulfate mit 10 - 16 C-Atomen in der Alkylgruppe und Alkylethersulfate mit 12 - 16 C-Atomen in der Alkylgruppe und 1 - 12 Glykolethergruppen. Weitere Aniontensid-Klassen, die in den erfindungsgemäßen Haarpflegemitteln enthalten sein können, sind Alkansulfonate mit 10 - 18 C-Atomen, Alken- und Hydroxyalkansulfonate, wie sie bei der Sulfonierung von α-Olefinen mit 10 - 18 C-Atomen erhalten werden, Fettsäurealkylolamid- und Fettsäurealkylolamidpolyglykolethersulfate, Fettsäuremonoglyceridsulfate, Sulfobernsteinsäuremonoalkylester, Acyltauride und Acylisethionate mit 10 - 18 C-Atomen in der Acylgruppe.

Haarpflegemittel können quartäre Ammoniumverbindungen der allgemeinen Formel (I) bis zu einer Menge von 50 Gew.-% des Aniontensids klar gelöst enthalten, ohne daß sich Trübungen oder Niederschläge bilden.

Wäßrige Lösungen, die 0,5 - 15 Gew.-% der quartären Ammoniumverbindungen der Formel (I) enthalten, lassen sich durch Zusatz von Elektrolyten in der Viskosität anheben. Auf diese Weise ist es möglich, klare wäßrige Haarpflegemittel, z.B. Haarnachspülmittel, herzustellen und diese ohne polymere Verdickungsmittel durch Zusätze von 0,1 - 10 Gew.-% eines einfachen Salzes aus der Gruppe der Chloride, Bromide, Sulfate, Carbonate und/oder Phosphate des Lithiums, Natriums, Kaliums oder Magnesiums auf eine für die Anwendung auf dem Haar geeignete Viskosität zu bringen. Es werden dabei strukturviskose Lösungen erhalten, die im Ruhestand höherviskos erscheinen als bei der unter Anwendung von Scherkräften erfolgenden Viskositätsmessung.

Als Elektrolyt zur Verdickung solcher Zubereitungen wird bevorzugt Natriumchlorid oder Magnesiumchlorid verwendet.

Eine bevorzugte Ausführungsform der Erfindung sind Haarpflegemittel in Form eines Haarwaschmittels

(Shampoos), gekennzeichnet durch einen Gehalt von

0,5 - 8 Gew.-% einer quartären Ammoniumverbindung der allgemeinen Formel (I) und
5 - 20 Gew.-% eines Aniontensids der allgemeinen Formel (II)
neben sonst üblichen Komponenten von Haarwaschmitteln.

Solche weiteren und bekannten Komponenten von Haarwaschmitteln sind z.B. Tenside anderer Ionogenität. Besonders bevorzugt ist die Verwendung einer Tensidkombination aus einem Aniontensid und einem ampholytischen oder zwitterionischen Tensid. Als ampholytische Tenside sind z.B. N-($C_8$-$C_{18}$)-Alkyl-ß-aminopropionsäuren oder N-Hydroxyethyl-N-kokosacylamidopropylglycin geeignet. Als zwitterionische Tenside (Betaintenside) können z.B. N-Kokosalkyl-dimethyl-glycin oder N-Kokosacylamido-propyl-dimethyl-glycin eingesetzt werden.

Auch nichtionogene Tenside können in untergeordneten Mengen in erfindungsgemäßen Shampoos enthalten sein. Geeignete nichtionogene Tenside sind z.B. Anlagerungsprodukte von 6 - 20 Mol Ethylenoxid an Fettalkohole mit 12 - 18 C-Atomen, an Fettsäuren mit 12 - 18 C-Atomen, an Alkylphenole mit 8 - 12 C-Atomen in der Alkylgruppe, an Fettsäurealkylolamide, an Fettsäuremono- und Diglyceride oder an Sorbitanfettsäureester. Weitere geeignete nichtionogene Tenside sind Fettsäuremono- und diethanolamide und Aminooxid-Tenside.

Daneben enthalten die erfindungsgemäßen Shampoos übliche Zusatz- und Stellmittel wie z.B. Verdikkungsmittel vom Typ der Fettsäurealkylolamide oder Polyvinylpyrrolidon, Trübungsmittel wie z.B. Ethylenglykoldistearat, pH-Wert-Stabilisatoren (Puffer) wie z.B. Alkali- oder Ammoniumphosphate oder Citrate, Konservierungsmittel, wie z.B. Formaldehyd, p-Hydroxybenzoesäureester, Farbstoffe, Duftstoffe sowie übliche haarkosmetische Wirkstoffe wie z.B. Antischuppenwirkstoffe, Sebostatika, Vitamine, Pflanzenextrakte usw.

Haarbehandlungsmittel in Form von Haarspülmitteln sind eine weitere bevorzugte Ausführungsform der Erfindung. Konditionierende Haarnachspülmittel werden üblicherweise als Öl-in-Wasser-Emulsion von Fettkomponenten (z.B. Cetylalkohol und/oder Stearylalkohol) in Wasser unter Zuhilfenahme nichtionogener Emulgatoren und Schutzkolloide formuliert. Als konditionierende Wirkstoffe sind quartäre Ammoniumverbindungen enthalten. Erfindungsgemäße Haarnachspülmittel können analog formuliert werden, wobei als quartäre Ammoniumverbindungen solche der allgemeinen Formel I eingesetzt werden.

Erfindungsgemäße Haarnachspülmittel können jedoch auch als klare, wäßrige Lösungen mit einem Gehalt von

0,5 - 10 Gew.-% der quartären Ammoniumverbindung der allgemeinen Formel (I) und
0,1 - 10 Gew.-% eines gelösten einfachen Salzes aus der Gruppe der Chloride, Bromide, Sulfate, Carbonate und/oder Phosphate des Lithiums, Natriums, Kaliums, Ammoniums oder Magnesiums
zubereitet werden.

Neben den kennzeichnenden Bestandteilen können die erfindungsgemäßen Haarnachspülmittel übliche Hilfsmittel wie z.B. Farbstoffe, Duftstoffe, Konservierungsmittel und haarkosmetische Wirkstoffe wie z.B. Antischuppenwirkstoffe, Sebostatika, Vitamine, Pflanzenextrakte usw. enthalten.

Erfindungsgemäße Haarnachbehandlungsmittel, die nach der Anwendung auf dem Haar verbleiben, wie z.B. Haarfestiger und Fönwellmittel, enthalten bevorzugt niedere Alkohole, insbesondere Ethanol und/oder Isopropanol in den in solchen Mitteln üblichen Mengen, dies sind für Festiger und Fönwellmittel 10 - 50 Gew.-%. Festiger und Fönwellmittel enthalten zusätzlich haarfestigende anionische und/oder nichtionogene filmbildende Polymere, z.B. Polyvinylpyrrolidone (z.B. Luviskol(R)) oder Copolymere aus Maleinsäure und Acryl- oder Methacrylsäureestern, in Mengen von 0,1 - 1,0 Gew.-%, bezogen auf das gesamte Mittel. Bei Aerosolpräparaten beziehen sich die genannten Prozentsätze auf die treibmittelfreie Zubereitung.

Die folgenden Beispiele sollen den Gegenstand der Erfindung näher erläutern ohne ihn hierauf zu beschränken.

## Beispiele

1. Haarkosmetische Prüfungen

Es wurden die erfindungsgemäßen Shampoos 1.1 und 1.2, die Vergleichsshampoos mit bekannten quartären Ammoniumverbindungen 1.3 und 1.4 und das Standardshampoo 1.5 ohne quartäre Ammoniumverbindung hergestellt und haarkosmetisch geprüft.

Folgende quartäre Ammoniumverbindungen (QAV) wurden verwendet:

1) 2-Hydroxyhexadecyl-2-hydroxyethyl-dimethyl-ammoniumchlorid (entspricht Formel I, wobei $R^1$ = n-Tetradecyl, $R^2$ = $R^4$ = $CH_3$, $R^3$ = $-CH_2-CH_2-OH$ und A = $Cl^-$)

2) 2-Hydroxyhexadecyl-bis-(2-hydroxyethyl)-methylammoniumchlorid (entspricht Formel I, wobei $R^1$ = n-Tetradecyl, $R^2$ = $-CH_3$ und $R^3$ = $R^4$ = $-CH_2-CH_2-OH$ und A = Cl )

3) Cetyl-trimethylammoniumchlorid (Dehyquart$^{(R)}$A)

4) Talgalkyl-tris-(oligooxyalkyl)-ammoniumphosphat (Dehyquart$^{(R)}$SP)

Die Zusammensetzung der Shampoos ist der Tabelle zu entnehmen.

Messung der Naßkämmbarkeit (Laborprüfung)

Es wurden standardisierte, unter definierten Bedingungen durch Blondierung und Kaltwelle vorgeschädigte Haarsträhnen verwendet. Diese wurden mit den in Tabelle 1 angegebenen Shampoos in handwarmem Wasser shampooniert und mit klarem Wasser nachgespült. Die Messung der Naßkämmbarkeit erfolgte durch eine Messung des Kämmwiderstandes, d.h. der Kraft, die erforderlich ist, um einen Kamm durch ein Haarbüschel zu ziehen. Dabei wurde eine modifizierte Zug-Prüfmaschine des Typs 1402 der Fa. Zwick (Einsingen in Ulm/Donau) verwendet. Die Prüfungsanordnung ist beschrieben in "Riechstoffe, Aromen, Kosmetika" Nr. 12, (1977), Seite 325, Spalte 2 und 3.

Um die Fehler möglichst gering zu halten, wurden 15-fache Bestimmungen des Kämmwiderstandes mit jedem der zu prüfenden Shampoos durchgeführt und die Mittelwerte gebildet. Die gemessenen Kämmwiderstandsmittelwerte wurden in Prozent des Standards angegeben. Der Standard wurde bestimmt nach Shampoonieren mit dem Shampoo 1.5 ohne quartäre Ammoniumverbindung und Nachspülen mit klarem Wasser.

Die Ergebnisse der Prüfung sind der Tabelle zu entnehmen:

Prüfung der haarkosmetischen Eigenschaften (Studioprüfung)

Die Prüfung und Beurteilung der haarkosmetischen Eigenschaften erfolgte an 10 Versuchspersonen von unterschiedlicher Haarqualität im sog. Halbseitentest. Der Test wurde von einem geschulten Friseur durchgeführt. Der Halbseitentest dient zur vergleichenden Prüfung zweier Haarbehandlungsmittel oder eines Haarbehandlungsmittels und eines Standards an Versuchspersonen.

Es wurde jeweils eines der Shampoos gemäß 1.1, 1.2, 1.3 und 1.4 mit dem Standardshampoo 1.5 verglichen. Die in der Tabelle aufgeführten Zahlen sind ein Maß dafür, wie sehr die Shampoos Nr. 1.1 - 1.4 von der Beurteilung des Standards Nr. 1.5 abweichen.

Durchführung und Auswertung

Das Haar der Versuchspersonen wurde angefeuchtet und in der Mitte gescheitelt. Dann wurde bei fünf der Versuchspersonen auf die linke Kopfhälfte das Standardshampoo 1.5 auf die rechte Kopfhälfte das Vergleichsshampoo in gleicher Menge von je 5 g appliziert (Vorwäsche). Bei den anderen fünf Versuchspersonen wurde jeweils die andere Kopfhälfte mit dem gleichen Produkt behandelt. Beide Seiten wurden in gleicher Weise shampooniert und mit Wasser gespült. Für die Hauptwäsche wurden noch einmal je 2.5 g der Shampoos auf die gleiche Kopfhälfte aufgetragen, eingeschäumt und mit klarem Wasser gründlich ausgespült.

Während und nach der Haarwäsche wurden die in der nachfolgenden Tabelle aufgeführten Kriterien durch den Friseur für jede der beiden Kopfhälften separat beurteilt. Die Beurteilung erfolgte durch die Vergabe von Noten (1 = ausgezeichnet, 2 = gut, 3 = mässig, 4 = schlecht). Aus den Noten der 10 Versuchspersonen für das gleiche Produkt wurden die Mittelwerte gebildet. Die Mittelwerte für das Standardprodukt wurden von den Mittelwerten für das Vergleichsprodukt subtrahiert. Die so erhaltene Paardifferenz der Mittelwerte der Beurteilungen von 10 Versuchspersonen wurde in die nachfolgende Tabelle eingetragen.

## Tabelle

| Prüfshampoos | 1,1 | 1,2 | 1,3 | 1,4 | 1,5 |
|---|---|---|---|---|---|
| | Gew.% | Gew.% | Gew.% | Gew.% | Gew.% |
| Fettalkohol ($C_{12-14}$)+ 2EO-sulfat-Na-Salz (28 %ig) | 50 | 50 | 50 | 50 | 50 |
| Q A V 1 | 2 | - | - | - | - |
| Q A V 2 | - | 2 | - | - | - |
| Dehyquart(R) A (25%ig) | - | - | 8 | - | - |
| Dehyquart(R) SP (50%ig) | - | - | - | 4 | - |
| Wasser (vollentsalzt) | 48 | 48 | 42 | 46 | 50 |
| Laborprüfung Naßkämmbarkeit (% Kämmwiderstand) | 83 | 72 | 88 | 120 | 100 |
| **Studioprüfung** nasses Haar | | | | | |
| Kämmbarkeit | 0,6 | 0,7 | 0,4 | 0,2 | 0 |
| Griff | 0,6 | 0,7 | 0,1 | 0,1 | 0 |
| **Studioprüfung** trockenes Haar | | | | | |
| Kämmbarkeit | 0,5 | 0,5 | -0,1 | 0 | 0 |
| Antistatik/Aufladbarkeit | 0,5 | 0,5 | -0,1 | 0 | 0 |
| Griff | 0,6 | 1,0 | -0,2 | 0 | 0 |

## 2. Anwendungsbeisiele

## 2.1 Haarnachspülmittel

| | 2.1.1 | 2.1.2 | 2.1.3 |
|---|---|---|---|
| QAV 1 | 3 Gew.% | - | - |
| QAV 2 | - | 2,5 Gew.% | - |
| QAV 2 | - | - | 5 Gew.% |
| Natriumchlorid | 7 Gew.% | 6 Gew.% | 5 Gew.% |
| Citronensäure | bis pH=6,5 | bis pH=6,5 | b.pH=6,5 |
| Wasser | ad 100% | | ad 100% |
| Viskosität (20°C) m Pa·s | 100 | 100 | 400 |

## 2.2 Haarshampoos

| | 2.2.1 | 2.2.2 | 2.2.3 | 2.2.4 | 2.2.5 |
|---|---|---|---|---|---|
| | Gew.% | Gew.% | Gew.% | Gew.% | Gew.% |
| ($C_{12/14}$)-Fettalkohol -2EO-sulfat-Na-salz (28 %ig) | 35 | 40 | 50 | - | - |
| ($C_{12/14}$)-Fettalkohol-sulfat-triethanolammoniumsalz (42 %ig) | - | - | - | 30 | - |
| $C_{12/14}$)-Fettalkohol-sulfat -monoethanol-ammoniumsalz (32 %ig) | - | - | - | - | 30 |
| QAV 1 | 1 | - | 1,5 | - | - |
| QAV 2 | - | 2,5 | - | 2 | 2 |
| Konservierungsmittel (Bronidox L) | 0,2 | 0,2 | 0,2 | 0,2 | 0,2 |
| Citronensäure bis pH= | 6,5 | 6,5 | 6,5 | 6,5 | 6,5 |
| Wasser (vollentsalzt) | ad 100% | ad100% | ad100% | ad100% | ad100% |

## Ansprüche

1. Haarpflegemittel mit einem Gehalt an quartären Ammoniumverbindungen, dadurch gekennzeichnet, daß als quartäre Ammoniumverbindungen solche der allgemeinen Formel (I)

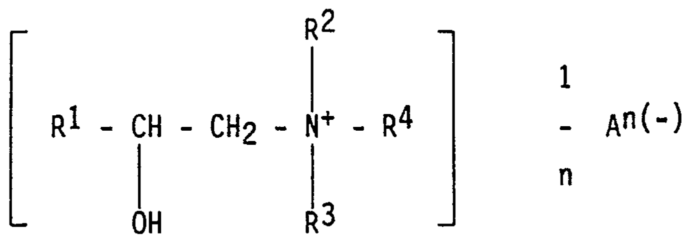

(I),

in der $R^1$ eine n-Alkylgruppe mit 12 - 16 C-Atomen, $R^2$ eine n-Alkylgruppe mit 1 - 4 C-Atomen, $R^3$ eine Hydroxyalkylgruppe mit 2 - 4 C-Atomen und $R^4$ eine n-Alkylgruppe mit 1 - 4 C-Atomen oder eine Hydroxyalkylgruppe mit 2 - 4 C-Atomen, A ein anorganisches oder organisches Säureanion und n dessen Wertigkeit darstellen, enthalten sind und das Mittel neben üblichen Zusätzen solcher Mittel entweder

a) Aniontenside der allgemeinen Formel (II)

$$R^5\text{-}O(C_2H_4O)_n\text{-}SO_3\ M \qquad (II),$$

in der $R^5$ eine lineare Alkylgruppe mit 10 - 16 C-Atomen, n = 0 oder eine Zahl von 1 - 12, M ein Lithium-, Natrium-, Kalium-, Magnesium-, Ammonium-, Mono-, Di- oder Triethanolammonium darstellt,

oder

b) gelöste einfache Salze aus der Gruppe der Chloride, Bromide, Sulfate, Carbonate und/oder Phosphate des Lithiums, Natriums, Kaliums oder Magnesiums enthält.

2. Haarpflegemittel nach Anspruch 1, dadurch gekennzeichnet, daß $R^2$ eine Methylgruppe, $R^3$ eine 2-Hydroxyethylgruppe, $R^4$ eine Methyl- oder 2-Hydroxyethylgruppe und A ein Chloridanion darstellen.

3. Haarpflegemittel nach Anspruch 1 oder 2 in Form eines Haarwaschmittels, gekennzeichnet durch einen Gehalt von

| | |
|---|---|
| 0,5 - 8 Gew.-% | der quartären Ammoniumverbindung der allgemeinen Formel (I) und |
| 5 - 20 Gew.-% | eines Aniontensids der allgemeinen Formel (II) |
| | neben sonst üblichen Komponenten von Haarbehandlungswaschmitteln. |

4. Haarpflegemittel nach Anspruch 1 oder 2, dadurch gekennzeichnet, daß dieses als klare wäßrige Zubereitung vorliegt und einen Gehalt von

| | |
|---|---|
| 0,5- 15 Gew.-% | der quartären Ammoniumverbindung der allgemeinen Formel (I) und |
| 0,1- 10 Gew.-% | eines gelösten einfachen Salzes aufweist. |

## Claims

1. Hair-care prepartions containing quaterary ammonium compounds, characterized in that the quaternary ammonium used correspond to general formula (I)

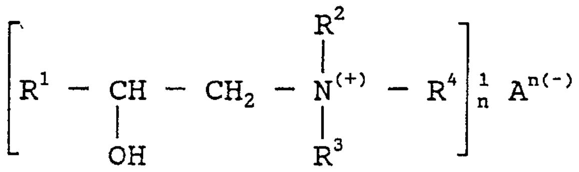 (I)

in which $R^1$ is a $C_{12-16}$ n-alkyl group, $R^2$ is a $C_{1-4}$ n-alkyl group, $R^3$ is a $C_{2-4}$ hydroxyalkyl group and $R^4$ is a $C_{1-4}$ n-alkyl group or a $C_{2-4}$ hydroxyalkyl group, A is an inorganic or organic acid anion and n is its valency, and, in addition to standard additives, the preparation contains either

a) anionic surfactants corresponding to general formula (II)

$$R^5{-}O(C_2H_4O)_n{-}SO_3M \qquad (II)$$

in which $R^5$ is a linear alkyl group containing 10 to 16 carbon atoms, n = 0 or a number of 1 to 12, M is a lithium, sodium, potassium, magnesium, ammonium, mono- di- or triethanolammonium,

or

b) dissolved simple salts from the group of chlorides, bromides, sulfates, carbonates and/or phosphates of lithium, sodium, potassium or magnesium.

**2.** Hair-care preparations as claimed in claim 1, characterized in that $R^2$ is a methyl group, $R^3$ is a 2-hydroxy ethyl group, $R^4$ is a methyl or 2-hydroxyethyl group and A is a chloride anion.

**3.** Hair-care preparations as claimed in claim 1 or 2 in the form of a shampoo, characterized by a content of

| | |
|---|---|
| 0.5 to 8% by weight | of a quaternary ammonium compound corresponding to general formula I and |
| 5 to 20% by weight | of an anionic surfactant corresponding to general formula II, in addition to other standard components of shampoos. |

**4.** A hair-care preparation as claimed in claim 1 or 2, characterized in that it is formulated as a clear aqueous preparation and contains

| | |
|---|---|
| 0.5 to 15% by weight | of the quaternary ammonium compound corresponding to general formula I and |
| 0.1 to 10% by weight | of a dissolved simple salt. |

## Revendications

**1.** Produit pour soigner les cheveux, renfermant des composés d'ammonium quaternaire, caractérisé en ce que sont contenus comme composés d'ammonium quaternaire, ceux de la formule générale (I)

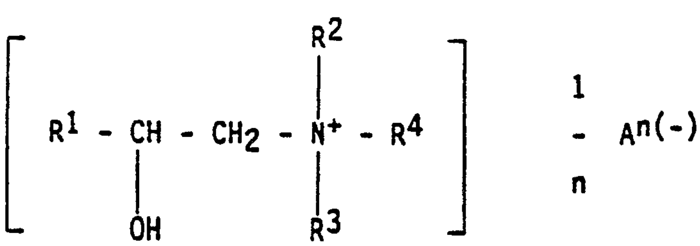 (I),

dans laquelle $R^1$ représente un groupement n-alkyle comportant 12 à 16 atomes de C, $R^2$ est un groupement n-alkyle comportant 1 à 4 atomes de C, $R^3$ correspond à un groupement hydroxyalkyle comportant 2 à 4 atomes de C, et $R^4$ représente un groupement n-alkyle comportant 1 à 4 atomes de C ou un groupement hydroxyalkyle comportant 2 à 4 atomes de C, A correspond à un anion d'acide

inorganique ou organique et n représente sa valence, et en ce que le produit contient, en plus des additifs usuels de tels produits, soit

a) des tensioactifs anioniques de la formule générale (II)

$$R^5-O(C_2H_4O)n-SO_3\ M \qquad (II)$$

dans laquelle $R^5$ représente un groupement alkyle linéaire comportant 10 à 16 atomes de C, n est égal à 0 ou à un nombre de 1 à 12, M correspond à un atome de lithium, sodium, potassium, magnésium ou à un groupe ammonium, mono-, di ou triéthanolammonium,

soit

b) des sels simples dissous faisant partie du groupe des chlorures, bromures, sulfates, carbonates et/ou phosphates de lithium, sodium, potassium ou magnésium.

**2.** Produit pour soigner les cheveux selon la revendication 1, caractérisé en ce que $R^2$ représente un groupement méthyle, $R^3$ est un groupement 2-hydroxyéthyle, $R^4$ correspond à un groupement méthyle ou à un groupement 2-hydroxyéthyle et A représente un anion chlorure.

**3.** Produit pour soigner les cheveux selon la revendication 1 ou 2, sous la forme d'un produit pour le lavage de la chevelure, caractérisé en ce qu'il contient:

0,5 à 8 % en poids de composé d'ammonium quaternaire de la formule générale (I) et
5 à 20 % en poids d'un tensioactif anionique de la formule générale (II).

à côté de constituants sinon usuels dans les produits pour le lavage des cheveux.

**4.** Produit pour soigner les cheveux selon la revendication 1 ou 2, caractérisé en ce que celui-ci se présente sous la forme d'une préparation aqueuse limpide et contient

0,5 à 15 % en poids de composé d'ammonium quaternaire de la formule générale (I) et
0,1 à 10 % en poids d'un sel simple dissous.